# EUROPEAN PATENT APPLICATION

(11) **EP 1 500 392 A1**
(43) Date of publication of application: **26.01.2005**
(21) Application number: 04017332.0
(22) Date of filing: 22.07.2004
(51) Int. Cl.: A61K 31/222, A61K 31/137, A61P 9/10

(54) **Aminotetralin derivatives as a medicament for the treatment and prevention of myocardial ischemic conditions**

(30) Priority: 24.07.2003 EP 03016873
(71) Applicant: CHIESI FARMACEUTICI S.p.A., I-43100 Parma (IT)
(72) Inventor: Rossoni, Giuseppe Battista, 43100 Parma (IT); Razzetti, Roberta, 43100 Parma (IT)
(74) Representative: Minoja, Fabrizio, Dr.

(57) **Abstract**

The use of 5,6-dihydroxy-2-methylaminotetralin (CHF 1035) and of 5,6-diisobutyroyloxy-2-methylaminotetralin (CHF 1024) for the preparation of a medicament for the treatment and prevention of myocardial ischemic conditions.

## Description

The invention concerns the use of aminotetralin derivatives for the preparation of medicaments for the treatment and prevention of myocardial ischemic conditions.

### BACKGROUND OF THE INVENTION

Systolic function of the heart is governed by four major determinants: the contractile state of the myocardium, the preload of the ventricle (the end-diastolic volume and the resultant fiber length of the ventricles prior to onset of the contraction), the afterload applied to the ventricles (the impedance to left ventricular ejection) and the heart rate. Alterations in any of these determinants may impair cardiac function and manifestations of cardiac failure occur.

Myocardial failure is characterized by two hemodynamic derangements, and the clinical presentation is determined by their severity. The first is reduction in cardiac reserve, i.e., the ability to increase cardiac output in response to increased demands imposed by exercise or even ordinary activity. The second abnormality, elevation of ventricular diastolic pressures, is primarily a result of the compensatory processes.

The progress in the understanding of the pathophysiologic features of the heart failure has led to the development of multiple therapeutic interventions including angiotensin-converting enzyme (ACE) inhibitors and, most recently, β-blockers and other neurohormonal antagonists. The syndrome is usually a progressive process resulting in geometry change of the left ventricle (cardiac remodeling) with increase in haemodynamic stresses of the walls of the failing heart, and depression of its mechanical performance.

The aminotetraline derivatives 5,6-dihydroxy-2-methylaminotetralin (CHF 1035) and its active metabolite 5,6-diisobutyroyloxy-2-methylaminotetralin (CHF 1024),disclosed in GB 2123410 are endowed with selective agonist activity on DA₂-dopaminergic and α₂-adrenergic receptors.

WO 96/29065 discloses their use in the preparation of a medicament for the therapy of congestive heart failure whereas WO 00/76544 discloses their use in treating left ventricular remodeling.

### DESCRIPTION OF THE INVENTION

It has now been found that CHF 1035 and its active metabolite CHF 1024 are endowed with myocardial antiischemic properties and are useful for the preparation of a medicament for the treatment and prevention of myocardial ischemic conditions.

Myocardial ischemia is a condition in which oxygen deprivation to the heart muscle is accompanied by inadequate removal of metabolites because of reduced blood flow or perfusion. During ischemia, an imbalance occurs between myocardial oxygen supply and demand. Myocardial ischemia can occur as a result of increased myocardial oxygen demand, reduced myocardial oxygen supply, or both. In many circumstances, it results from both an increase in oxygen demand and a reduction in supply.

A study was carried out utilizing isolated perfused rat heart according to the method described in *J Cardiovasc Pharmacol 1998; 32: 260-5.*

In this model, CHF 1024 has demonstrated a dose-dependent antiischemic activity, which was particularly marked at 100 nM. It was supported by concomitant and noticeable reduction of creatine kinase (CK) and lactate dehydrogenase (LDH) enzymes released in the perfusate during heart reperfusion with consequent decrease in both heart stiffness and coronary perfusion pressure (CCP).

Indeed, CHF-1024 dose-dependently inhibits ventricular contracture during ischemia and significantly improves left ventricular developed pressure (LVDP) during reperfusion, being at this time left ventricular end-diastolic pressure (LVEDP) diminished as compared to vehicle-treated preparations. Another finding in favor of the cardioprotective effect of CHF-1024 is the maintenance of higher rate of 6-Keto-prostaglandin F_{1α} (6-Keto-PGF_{1αα}) release during reperfusion, being the PGI₂ production in the heart during reperfusion a critical cytoprotective mechanism for resisting the damage caused by ischemia.

For the considered therapeutic use, CHF 1035 or CHF 1024 will be administered at dosages which will be easily determined and adjusted by the skilled practitioner, by any administration route, the oral, parenteral or transdermal route being particularly preferred. Generally, daily dosages ranging from 0.01 to 1 mg/kg/die, and for example, particularly in case of oral administration, unit daily dosages from 2.5 to 100 mg, preferably from 9.5 to 50 mg, even more preferably from 5 to 25 mg can be envisaged.

Said compounds may optionally be administered in combination with other drugs known to be beneficial for the considered treatment. Examples of suitable administration forms include capsules, tablets, injectable sterile solutions or suspensions, transdermal ointments or gels and the like.

The invention will be described in more details hereinafter, by means of the annexed Figures and experimental tests.

### DESCRIPTION OF THE FIGURES

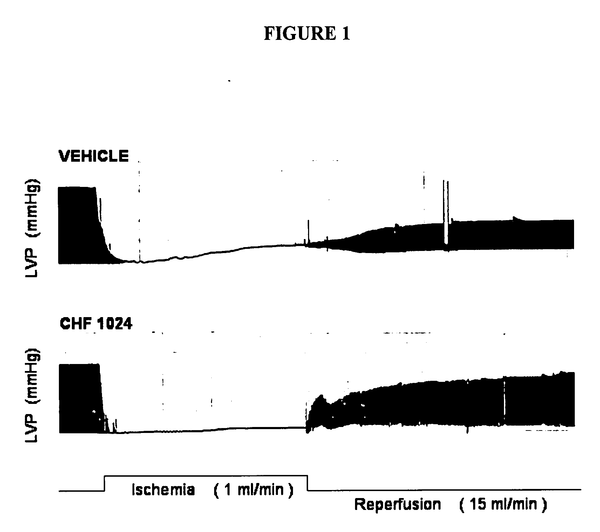

**Figure 1** Two representative ischemia-reperfusion tracings obtained from the perfused rat hearts treated with vehicle (upper panel) or 100 nM CHF-1024 (lower panel). Vehicle or CHF-1024 were perfused through the hearts for the final 15 min of pre-ischemia.

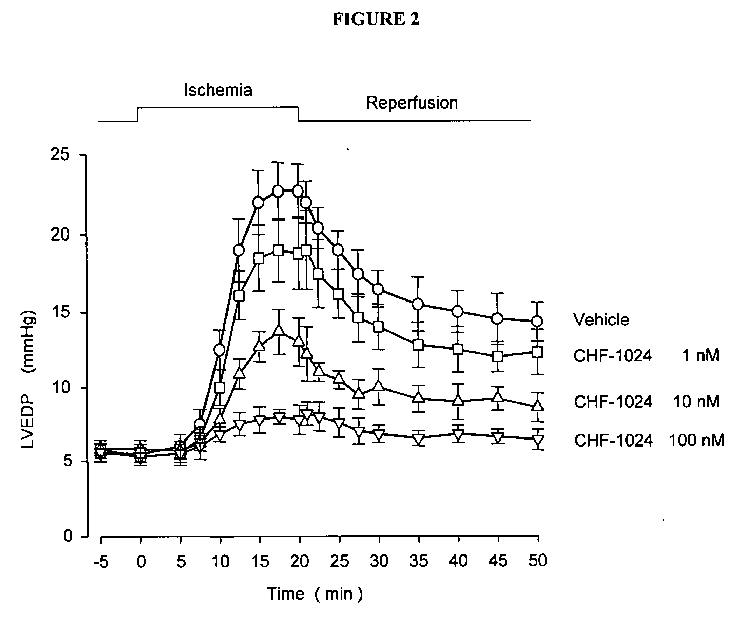

**Figure 2** Left ventricular end-diastolic pressure (LVEDP) in isovolumic left rat heart preparations submitted to 20-min low flow ischemia (1 ml/min) and 30-min reperfusion (15 ml/min). Values are mean ± S.E.M. of 8 different hearts for each group. Vehicle or CHF-1024 were perfused for 15 min before ischemia. For statistical comparisons see the area under the curve values reported in Table 1.

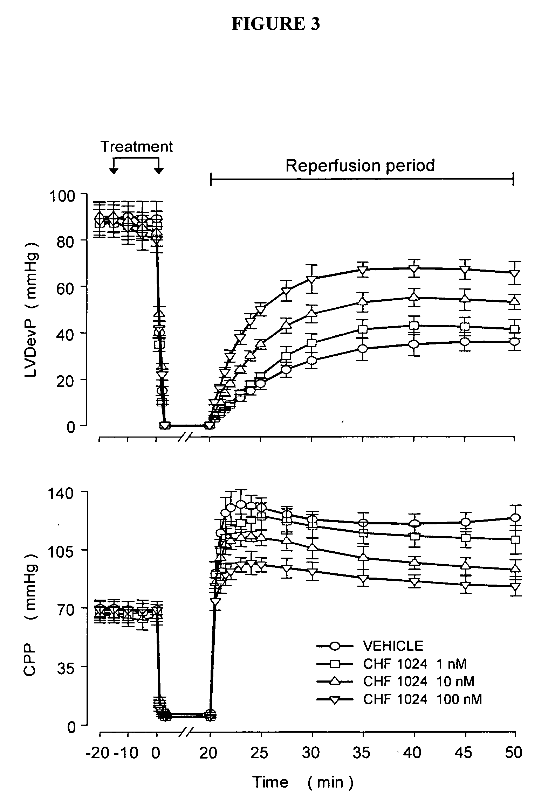

**Figure 3** Left ventricular developed pressure (LVDevP) and coronary perfusion pressure (CPP) in isovolumic left rat heart preparations submitted to 20-min low flow ischemia (1 ml/min) and 30-min reperfusion (15 ml/min). Values are mean ± S.E.M. of 8 different hearts for each group. Vehicle or CHF-1024 were perfused for 15 min before ischemia. For statistical comparisons see the area under the curve values reported in Table 1.

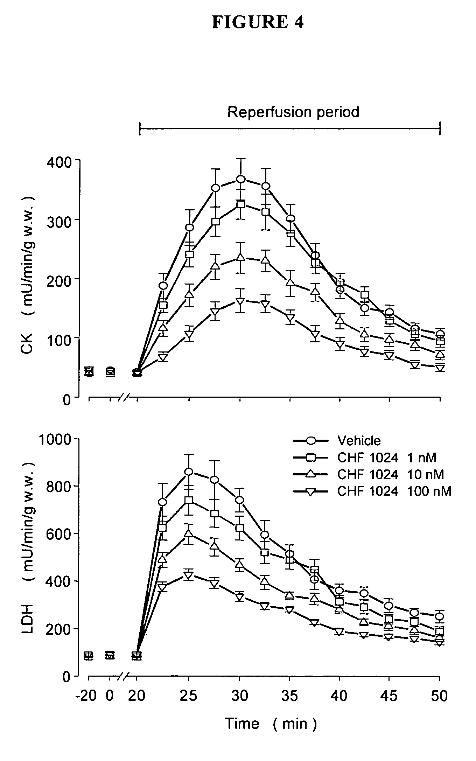

**Figure 4** Creatine kinase (CK) and lactate dehydrogenase (LDH) release profile in isovolumic left rat heart preparations submitted to 20-min low flow ischemia (1 ml/min) and 30-min reperfusion (15 ml/min). Values are means ± S.E.M. of 8 different hearts for each group. Vehicle or CHF-1024 were perfused for 15 min before ischemia. For statistical comparisons see the area under the curve values reported in Table 1.

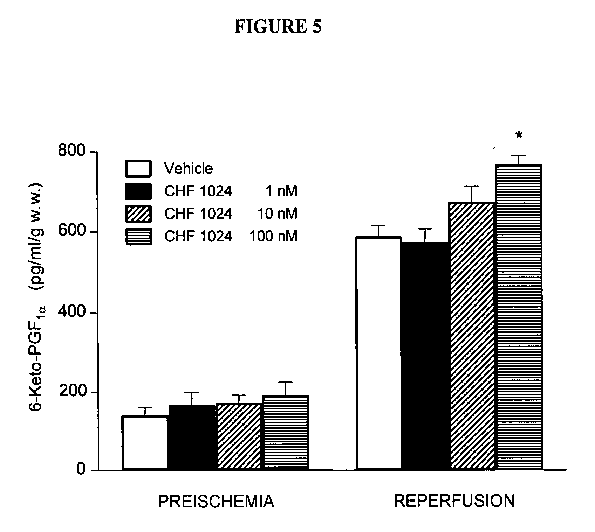

**Figure 5** Rate of 6-Keto-PGF_{1α} formation in isovolumic left rat heart preparations submitted to 20-min low flow ischemia (1 ml/min) and 30-min reperfusion (15 ml/min). Values are mean ± S.E.M. of 8 different hearts for each group. Perfusates were collected before ischemia (preischemia) and during the first 10 min of reperfusion. Values are the means ± SEM of 8 different hearts for each group. **p*<0.05 versus vehicle-treated preparations in the reperfusion period.

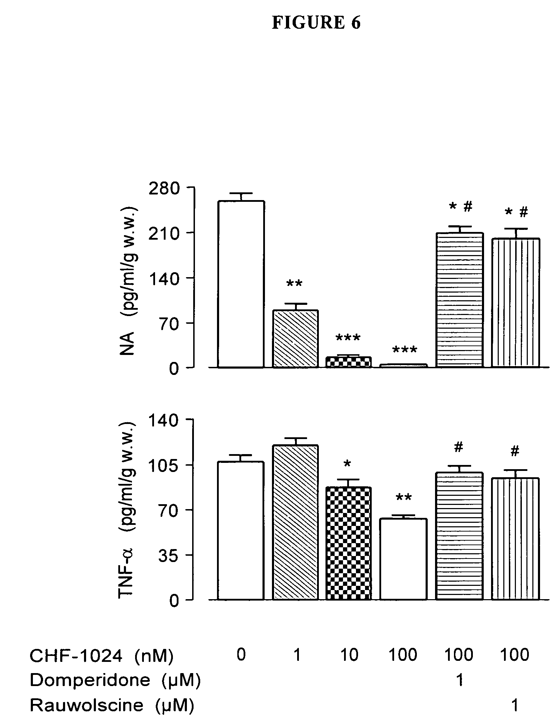

**Figure 6** Release of noradrenaline (NA; upper panel) and tumor necrosis factor-α (TNF-α) in the perfusates collected in the first 2 min during heart reperfusion. *p<0.05, **p<0.01 and ***p<0.001 versus vehicle-treated hearts; ^{#}p<0.001 versus 100 nM CHF-1024-treated preparations.

### EXPERIMENTAL TESTS

Male Wistar rats were used in the present study.

### Perfusion of rat heart

Perfusion of rat heart was performed by the method described previously *(J Cardiovasc Pharmacol* 1998;32:260-5). In brief, the rats were anaesthetized with sodium pentobarbital (60 mg/kg) given by intraperitoneal injection. The chest were opened, and the heart was rapidly excised, and placed in cold (4°C) heparinized (20 units/ml) Krebs Henseleit solution. The heart was mounted within 2 min after thoracotomy on the experimental setup and perfused retrogradely at 15 ml/min via the aorta with Krebs Henseleit solution, which was maintained at 37°C and aerated with 95% O₂/5% CO₂ to pH 7.4. Coronary perfusion pressure (CPP), left ventricular pressure (LVP) and left ventricular developed pressure (LVDevP; peak left ventricular systolic pressure minus LVEDP) were measured.

### Ischemia/reperfusion

After equilibration of 15 min, hearts were paced at 300 beats/min with an electrical stimulator *via* two silver electrodes attached to the right atrium and an additional 30 min of perfusion was carried out (pre-ischemic period). Ischemia was induced by reducing the flow rate from 15 ml/min to 1 ml/min for 20 min (ischemic period). A normal flow rate (15 ml/min) was then restored and the perfusion was continued for another 30 min (reperfusion period).

In groups of 8 hearts each, CHF-1024 (1, 10 and 100 nM) was perfused through the hearts for the final 15 min of pre-ischemia period.

Furthermore, in separate experiments (6 hearts for each group), 100 nM CHF-1024 was perfused during pre-ischemic period in combination with the DA₂-dopaminergic and α₂-adrenergic receptor antagonists domperidone (1 µM) or rauwolscine (1 µM), respectively. In these hearts the two antagonists were administered upstream of the coronary bed for 10 min (15-min before CHF-1024) by a microdialysis pump with an infusion rate adjusted to 1/75^{th} of the coronary flow.

Creatine kinase (CK) and lactate dehydrogenase (LDH) activities in heart perfusates

The perfusate, eluted from the heart during pre-ischemic and reperfusion periods, was collected in an ice-cooled beaker as 2.5-min samples collection. Each sample was used for the determination of CK and LDH activities by means of a spectrophotometrical method..

### Prostacyclin (PGI₂) determination in heart perfusates

PGI₂ was measured directly in the coronary effluent collected in an ice-cooled beaker for 10 min immediately before ischemia and during the first 10 min of reperfusion. PGI₂ was determined as its stable metabolite 6-Keto-prostaglandin F_{1α} (6-Keto-PGF_{1α}) according to the enzyme-linked immunosorbent assay.

### Noradrenaline (NA) and tumor necrosis factor-alpha (TNF-α) determinations in heart perfusates

NA and TNF-α were measured in the coronary effluent collected during the last 2 min of the pre-ischemic period and over the first 2 min of the reperfusion period in an ice-cooled beaker as 30 sec samples-collection.

After isolation by the alumina adsorption method, NA concentration was determined by the high-pressure liquid chromatography (HPLC) technique. The concentrations of NA was expressed in pg/ml/g wet heart weight (pg/ml/g w.w.).

TNF-α was assessed with an ELISA-kit. The concentrations of TNF-α were expressed in pg/ml/g wet heart weight (pg/ml/g w.w.).**RESULTS**

### Influence of CHF-1024 on the perfused isolated heart

The time course of changes in LVDevP, LVEDP and CPP of ischemic-reperfused rat hearts treated with 1, 10 or 100 nM CHF-1024 are depicted in Fig. 1-2-3 and summarized as AUC values in Table 1. Addition of CHF-1024 to the perfusion medium for 15 min before ischemia had no effect on LVEDP or CPP and only at the high concentration (100 nM) CHF-1024 slightly depressed LVDevP (-9%; p > 0.05 vs. baseline) (Fig. 2-3).

During ischemic period, the values of LVEDP of the vehicle-treated hearts after standstill began progressively to rise reaching the peak approximately at 15 min (from 5.5 ± 0.4 to 22.7 ± 1.3 mm Hg; p<0.001). However, LVEDP slightly declined during reperfusion, and at the end of this period was still significantly elevated (14.3 ± 0.8 mm Hg; p<0.01 vs. pre-ischemic value) (Fig. 2). Treatment with 1, 10 or 100 nM CHF-1024 before ischemia attenuated the rise in LVEDP during ischemic and reperfusion periods in a concentration-dependent manner (Fig. 1-2 and Table 1).

In vehicle-treated hearts, LVDevP during reperfusion was significantly depressed and at the end of this period the heart contractility recovered only 40% (36 ± 4 mm Hg) of the pre-ischemic values (90 ± 6.5 mm Hg) (Fig. 3). In hearts treated with various concentrations of CHF-1024 (1, 10 or 100 nM), LVDevP was partially recovered in a concentration-dependent manner at the end of reperfusion. In this instance, CHF-1024 at 10 and 100 nM caused a recovery of LVDevP of 64 ± 5% and 82 ± 7% (p<0.01 vs. vehicle-treated group) of the pre-ischemic value, respectively (Fig. 1-3 and Table 1).

The CPP of the vehicle-treated heart increased upon reperfusion and reached the maximum level at 3 min indicating both stiffness of the ventricular myocardium, and coronary vasoconstriction. These effects observed during reperfusion were reduced in a concentration-dependent manner in hearts treated with 1, 10 or 100 nM CHF-1024 (Fig. 3 and Table 1).

Since treatment with 100 nM CHF-1024 resulted in the maximum recovery of heart mechanics and CPP, the same dosage was used in separate experiments to characterize the cardioprotective activity of this compound. Pre-treatment of the hearts for 10 min in the pre-ischemic period (15-min before 100 nM CHF-1024) with 1 µM domperidone or 1 µM rauwolscine significantly reduced the cardioprotective activity of CHF-1024 on ischemia-reperfusion injury. As shown in Table 1, the AUC values related to LVEDP, LVDevP and CPP obtained with the combination of domperidone or rauwolscine plus CHF-1024 were significantly different from those shown in the hearts treated with CHF-1024 alone.

**Table 1**

| | | | | |
|---|---|---|---|---|
| *Paced isovolumic left rat heart preparations subjected to 20-min low flow ischemia and 30-min reperfusion: area under the curve (AUC) values related to left ventricular end-diastolic pressure (LVEDP), left ventricular developed pressure (LVDevP) and coronary perfusion pressure (CPP)* | | | | |

| **Treatment** | **No. exp.** | **AUC related to:** | | |
|---|---|---|---|---|
| | | **LVEDP** | **LVDevP** | **CPP** |
| Vehicle | 8 | 492 ± 52 | 908 ±52 | 1648 ± 122 |
| CHF-1024 1 nM | 8 | 384 ± 46 | 1031 ±86 | 1424 ± 89 |
| CHF-1024 10 nM | 8 | 205 ± 27^{*b*} | 1423 ± 77^{*a*} | 1106 ± 95^{*a*} |
| CHF-1024 100 nM (CHF) | 8 | 96 ± 12^{*b*} | 1790 ± 124^{*b*} | 623 ± 51^{*b*} |
| | | | | |
| Domperidone 1 µM (D) | 6 | 475 ± 48 | 875 ± 48 | 1726 ± 104 |
| Rauwolscine 1 µM (R) | 6 | 505 ± 64 | 912 ± 64 | 1680 ± 98 |
| D + CHF | 6 | 327 ± 41^{*c*}^{,}^{*d*} | 1327 ± 81^{*cd*} | 1243 ± 68^{*c*}^{,}^{*d*} |
| R + CHF | 6 | 353 ± 33^{*c*}^{,}^{*d*} | 1373 ± 103^{*c*}^{,}^{*d*} | 1260 ± 81^{*c*}^{,}^{*d*} |

Data are mean ± S.E.M. The AUC values were estimated according to the trapezoid method: in ordinate, LVEDP, LVDevP or CPP in mm Hg; in abscissa, time from 0 to 50 min (ischemic plus reperfusion periods) for LVEDP or time from 20 to 50 min (reperfusion period) for LVDevP and CPP.

Statistical differences: ^{*a*}p<0.01 and ^{*b*}p<0.001 vs. vehicle; ^{*c*}p<0.05 vs. D or R alone; ^{*d*}p<0.001 vs. CHF alone.

### CK and LDH activities in heart perfusates

CK and LDH, two indicators of myocardial damage, were determined in the coronary effluent collected from each heart in a 2.5-min sample during pre-ischemic and reperfusion periods. As shown in Fig. 4, there were no differences among the various groups of hearts in CK and LDH release during the pre-ischemic period. However, during 30-min reperfusion CK and LDH activities measured in vehicle-treated groups were 8.7-fold and 9.6-fold higher (p<0.001) than those found in pre-ischemic period, respectively (Fig. 4). Treatment with 1, 10 or 100 nM CHF-1024 significantly reduced in a concentration-dependent manner the CK and LDH release at the reperfusion as compared to vehicle-treated hearts (Fig. 4 and Table 2). Pre-treatment of the hearts with domperidone or rauwolscine attenuated the effect of CHF-1024 on CK and LDH release from reperfused heart (Table 2).

**Table 2**

| | | | |
|---|---|---|---|
| *Paced isovolumic left rat heart preparations subjected to low flow ischemia and reperfusion: area under the curve (A UC) value related to creatine kinase (CK) and lactate dehydrogenase (LDH) activities* | | | |

| **Treatment** | **No. exp.** | **AUC related to:** | |
|---|---|---|---|
| | | **CK** | **LDH** |
| Vehicle | 8 | 5617 ± 487 | 12673 ±750 |
| CHF-1024 1 nM | 8 | 4992 ±368 | 10729 ±887 |
| CHF-1024 10 nM | 8 | 3173 ± 215^{*a*} | 7836 ± 570^{*a*} |
| CHF-1024 100 nM (CHF) | 8 | 1695 ± 146^{*a*} | 4836 ± 390^{*a*} |
| | | | |
| Domperidone 1 µM (D) | 6 | 5815 ± 321 | 12795 ± 617 |
| Rauwolscine 1 µM (R) | 6 | 5584± 297 | 12988 ± 522 |
| D + CHF | 6 | 4070 ± 402^{*b*}^{,}^{*d*} | 8572 ± 827^{*b*}^{,}^{*c*} |
| R + CHF | 6 | 4132 ± 375^{*b*}^{,}^{*d*} | 9221 ± 994^{*b*}^{,}^{*d*} |

Data are mean ± S.E.M. The AUC values were estimated according to the trapezoid method: in ordinate, CK or LDH in mU/min/g w.w.; in abscissa, time from 20 to 50 min (reperfusion period).

Statistical differences: ^{*a*}p<0.001 vs. vehicle; ^{*b*}p<0.01 vs. D or R alone; ^{*c*}p<0.01 and ^{*d*}p<0.001 vs. CHF alone.

### PGI₂ release in heart perfusates

It is well known that PGI₂ is the major eicosanoid produced by jeopardized myocardium (*Mol Cell Biochem* 1989;88:113-21); the rate of formation of this lipidic material increases particularly during the first 5-10 min of reperfusion declining thereafter rapidly. In the present study, in vehicle treated hearts the generation of 6-Keto-PGF_{1α} (the stable metabolite of PGI₂) during reperfusion was 4.1-fold enhanced (587 ± 27 pg/ml/g w.w.; p<0.001) compared to that found in heart-perfusates in the pre-ischemic period (142 ± 20 pg/ml/g w.w.) (Fig. 5). When the hearts were perfused with CHF-1024 at the higher concentration (100 nM) for 15 min before ischemia, a further slight increase (1.3-fold; p<0.05 vs. vehicle-treated hearts) in 6-Keto-PGF_{1α} rate of formation was observed during reperfusion (Fig. 5).

### NA and TNF-α release in heart perfusates

In preliminary experiments, it was found that the bulk of NA outflow occurs within the first 2 min of reperfusion period, and thereafter it declines to baseline within 10-15 min (data not shown). NA release was almost undetectable in 2-min samples of coronary effluent taken immediately before ischemia (4.1 ± 0.5 pg/ml/g w.w.) but it was significantly elevated during the first 2 min of reperfusion (258 ± 16 pg/ml/g w.w.) (Fig. 6). CHF-1024 perfused for 15 min through the heart at 1, 10 or 100 nM in the pre-ischemic period significantly prevented the increase of NA outflow by 66% (p<0.01), 94% (p<0.001) and 98% (p<0.001), respectively, as compared to vehicle-treated hearts (Fig. 6).

Significant amount of TNF-α (107.1 ±5.8 pg/ml/g w.w.) was detected in the coronary effluent only during the first 2 min of reperfusion, since 10 min later its concentration was below the sensitivity of the assay (<10 pg/ml; data not shown) (Fig. 6). CHF-1024 given at different concentrations before ischemia prevented the rate of TNF-α release from the heart in a concentration-dependent manner. In particular, at 10 and 100 nM, CHF-1024 reduced by 19% (p<0.05) and 41 % (p<0.01 ) respectively the rate of TNF-α release as compared to the corresponding values of vehicle-treated hearts (Fig. 6).

In the experiments when perfusion with 100 nM CHF-1024 was preceded by 10-min infusion of domperidone (1 µM) or rauwolscine (1 µM), NA outflow during the first 2 min of heart-reperfusion was reduced only by 19% (p<0.05) and 23% (p<0.05) compared with the corresponding values of vehicle-treated hearts (Fig. 6).

Similar results were obtained for TNF-α release; in this case, the combined treatment of domperidone or rauwolscine plus 100 nM CHF 1024 resulted in a slight not significant reduction (p>0.05) of TNF-α release as compared to the vehicle-treated group (Fig. 6).

The results obtained with CHF-1024 in the present study clearly indicate that this compound displays beneficial effects against myocardial ischemia and reperfusion damage. This can be referred to the reduction of release of NA from sympathetic nerve endings and of TNF-α from cardiac tissues during reperfusion.

## Claims

1. The use of 5,6-dihydroxy-2-methylaminotetralin (CHF 1035) and of 5,6-diisobutyroyloxy-2-methylaminotetralin (CHF 1024) for the preparation of a medicament for the treatment and prevention of myocardial ischemic conditions.
